# EUROPEAN PATENT SPECIFICATION

(11) **EP 4 284 368 B1**
(45) Date of publication and mention of the grant of the patent: **11.12.2024**
(21) Application number: 22705635.5
(22) Date of filing: 01.02.2022
(51) Int. Cl.: A61K 31/4545, A61P 31/12, A61P 31/14, A61P 31/16

(54) **USE OF THE AXL INHIBITOR SLC-391 AS ANTIVIRAL THERAPEUTIC AGENT**
VERWENDUNG DES AXL INHIBITORS SLC-391 ALS ANTIVIRALEN THERAPEUTISCHEN WIRKSTOF
UTILISATION DE L'INHIBITEUR AXL SLC-391 EN TANT QU'AGENT ANTIVIRAL

(30) Priority: 01.02.2021 US 202163144453 P
(43) Date of publication of application: 06.12.2023
(73) Proprietor: SignalChem Lifesciences Corporation, Richmond, BC V6V 2J2 (CA)
(72) Inventor: ZHANG, Zaihui, Richmond, British Columbia V6V 2J2 (CA); YAN, Jun, Richmond, British Columbia V6V 2J2 (CA); XIE, Zhiwei, Richmond, British Columbia V6V 2J2 (CA)
(74) Representative: Mewburn Ellis LLP
(86) International application number: PCT/US2022/014783
(87) International publication number: WO 2022/165436

(56) References cited:
- US-A1- 2015 252 370
- RAMKUMAR K.: "1736P Elevated AXL expression following SARS-CoV-2 infection in non-small cell lung cancer", ANNALS OF ONCOLOGY, vol. 31, no. S4, 1 September 2020 (2020-09-01), NL, pages S1015 - S1016, XP055909841, ISSN: 0923-7534, DOI: 10.1016/j.annonc.2020.08.1800
- DITTMAR MARK ET AL: "Drug repurposing screens reveal FDA approved drugs active against SARS-Cov-2", BIORXIV, 19 June 2020 (2020-06-19), XP055802639, Retrieved from the Internet <URL:https://www.biorxiv.org/content/10.1101/2020.06.19.161042v1.full.pdf> [retrieved on 20210508], DOI: 10.1101/2020.06.19.161042
- SAKSHI PIPLANI ET AL: "Computationally repurposed drugs and natural products against RNA dependent RNA polymerase as potential COVID-19 therapies", ARXIV.ORG, CORNELL UNIVERSITY LIBRARY, 201 OLIN LIBRARY CORNELL UNIVERSITY ITHACA, NY 14853, 29 November 2020 (2020-11-29), XP081824287
- BHATTI JASVINDER SINGH ET AL: "Therapeutic Strategies in the Development of Anti-viral Drugs and Vaccines Against SARS-CoV-2 Infection", MOLECULAR NEUROBIOLOGY, SPRINGER US, NEW YORK, vol. 57, no. 11, 18 August 2020 (2020-08-18), pages 4856 - 4877, XP037254745, ISSN: 0893-7648, [retrieved on 20200818], DOI: 10.1007/S12035-020-02074-2
- ENCINAR JOSÉ ANTONIO ET AL: "Potential Drugs Targeting Early Innate Immune Evasion of SARS-Coronavirus 2 via 2?-O-Methylation of Viral RNA", VIRUSES, vol. 12, no. 5, 1 May 2020 (2020-05-01), CH, pages 525, XP055816779, ISSN: 1999-4915, DOI: 10.3390/v12050525
- COLAVITO SIERRA A.: "AXL as a Target in Breast Cancer Therapy", JOURNAL OF ONCOLOGY, vol. 2020, 14 February 2020 (2020-02-14), US, pages 1 - 15, XP055909857, ISSN: 1687-8450, Retrieved from the Internet <URL:http://downloads.hindawi.com/journals/jo/2020/5291952.xml> DOI: 10.1155/2020/5291952

## Description

### BACKGROUND

### Technical Field

The present disclosure provides an antiviral therapy using an AXL kinase inhibitor to treat viral infectious diseases including COVID-19.

### Description of the Related Art

AXL is a member of the TAM (Tyro3, AXL, and MER) family of receptor tyrosine kinases. It is a transmembrane receptor (molecular weight: 100 and 140 kDa) that contains an extracellular (N-terminal) domain and an intracellular (C-terminal) tyrosine kinase domain. The TAM family functions as homeostatic regulators in normal adult tissues and organ systems.

AXL activation can be mediated either by ligand-dependent or ligand-independent receptor dimerization. Growth arrest-specific protein 6 (Gas6) has been identified as the main ligand that binds the extracellular domain of AXL, leading to dimerization of the Gas6/AXI, complex and resulting in autophosphorylation of tyrosine residues on the intracellular tyrosine kinase domain of AXL. Phosphorylation of these intracellular domains subsequently triggers downstream signaling pathways including PI3K-AKT-mTOR, MEK-ERK, NF-κB, and JAK/STAT (Gay 2017; Wium 2018; Schoumacher and Burbridge 2017). AXL is a putative driver of diverse cellular processes that are critical for the development, growth, and spread of tumors, and immune modulation (Schoumacher and Burbridge 2017), making AXL a promising target for the development of anti-cancer therapeutics.

Furthermore, AXL is a key suppressor of the type I interferon response and is targeted by viruses to block the anti-viral immunity. AXL is known to be used by several different enveloped viruses including pox-, retro-, flavi-, arena-, filo-, and alpha-viruses (Shimojima 2006, Meertens 2012, Dowall 2016, Meertens 2017). Many enveloped viruses display the phospholipid phosphatidylserine on their membranes, through which they bind GAS6 and Protein S, which then binds to AXL. Viral particle binding via GAS6-AXI, potently activates signal transduction through its tyrosine kinase domain to suppress type I interferon (IFN) signaling and facilitate viral replication (Bhattacharyya 2013, Meertens 2017). AXL increases viral infection through two mechanisms, namely enhanced viral entry through "apoptotic mimicry" and suppression of anti-viral type I interferon responses.

AXL signaling suppresses viral-induced IFN responses via SOCS1/3 and TBK1 (Sharif 2006, Cruz 2019), leading to increased viral replication in infected cells and decreased and-viral defenses of neighboring cells (Huang 2015, Chen 2018, Strange 2019). AXL is a unique type I interferon response checkpoint. IFNR signaling induces AXL expression. AXL is a critical negative feedback regulatory mechanism for TLR-induced type I interferon (IFN) responses in myeloid (dendritic cells, macrophages), NK and tumor cells (Rothlin 2007, Lee 2019, Canadas 2018).

Therapeutic AXL receptor inhibition ameliorated pulmonary pathology resulting from primary viral infection in experimental models, including an important role for AXL within the lung (Shibata 2014). During primary respiratory syncytial virus (RSV) infection, AXL inhibition increased the number of IFN gamma-producing T cells and NK cells, suppressed RSV replication and whole lung levels of IL-4 and IL-13. The lethal effect of intrapulmonary H1N1 infection inflammation was reduced by AXL inhibition. AXL inhibition in infected mice increased the number of IFN-beta-producing macrophages and dendritic cells and suppressed neutrophil infiltration (Shibata 2014). AXL-null mice are resistant to ZIKA pathogenesis likely due to a combination of reduced virus entry and enhanced IFN responses (Hastings 2019), indicating a potential role for AXL inhibitors as therapeutics during viral infection.

With AXL known to be targeted by several different enveloped viruses (e.g. Ebola, Zika) to enter cells and dampen the viral immune response, AXL has been recognized as a good drug target for the treatment of the infection of a wide range of enveloped viruses including SARS-CoV-2. BMS-777607, an inhibitor for AXL, strongly inhibited the ligand-dependent activation of both AXL and MER in cultured cell assays, effectively blocking Gas6-triggered receptor activation at concentrations ranging from 30-3600 nM. Wild type bone marrow-derived dendritic cells treated with BMS-777607 were much less susceptible to virus infection than untreated cells (Bhattacharyya 2013). It has been demonstrated that another AXL inhibitor BGB324 (Bemcentinib) had effect against lethal Ebola virus infection in animal models (Dowall 2016). In preliminary in vitro studies, Bemcentinib was shown to protect cells from SARS-CoV-2-induced cytopathic effect. Subsequent studies validated the ability of Bemcentinib to potently inhibit SARS-CoV-2 infection of cells, supporting the potential use of the AXL inhibitor for the treatment of early SARS-CoV-2 infection, and its selection as the first candidate to be fast-tracked in a UK multi-center Phase II clinical trial for hospitalized COVID-19 patients. (BerGenBio, Press release 28Apr2020).

As disclosed in "AXL Promotes SARS-CoV-2 Infection of Pulmonary and Bronchial Epithelial Cells" (Wang 2020) tyrosine-protein kinase receptor AXL were found to specifically interact with SARS-CoV-2 S protein on the host cell membrane. When overexpressed in cells that do not highly express either AXL or ACE2, AXL promotes virus entry as efficiently as ACE2. Strikingly, deleting AXL, but not ACE2, significantly reduces infection of pulmonary cells by the SARS-CoV-2 virus pseudotype. Soluble human recombinant AXL, but not ACE2, blocks SARS-CoV-2 virus pseudotype infection in pulmonary cells. Taken together, their findings suggest AXL may play an important role in promoting SARS-CoV-2 infection of the human respiratory system and is a potential target in future clinical intervention strategies.

In further support of the role of the use of AXL inhibitors as anti-viral agents is a study that explored the global phosphorylation landscape of SARS-CoV-2 infection (Bouhaddou 2020). Phosphoproteomic analysis of SARS-CoV-2-infected Vero E6 cells revealed that host cellular pathways are hijacked by viral infection. SARS-CoV-2 infection promoted casein kinase II (CK2) and p39 MAPK activation, production of diverse cytokines, and shutdown of mitotic kinases, resulting in cell cycle arrest. In a screening study of 68 drugs and compounds, pharmacologic inhibition of p38, CK2, CDK, AXL, and PIKFYVE kinases were found to possess antiviral activity, representing potential COVID-19 therapies. Potent antiviral activity was observed for AXL inhibitor gilteritinib (IC50=0.807uM). Authors noted that AXL is known to regulate various intracellular pathways, including Ras/ERK, PI3K, and p38. During SARS-CoV-2 infection, p38 inhibition suppressed cytokine production and impaired viral replication, suggesting that inhibition of AXL, and subsequent suppression of p38 signal transduction, may target multiple mechanisms related to COVID-19 pathogenesis.

Infection of lung cancer cells with SARS-CoV-2 has been found to induce metabolic and transcriptional changes consistent with epithelial to mesenchymal transition (EMT), including upregulation of ZEB1 and AXL, thereby downregulating ACE2 post-infection (Stewart 2020). Researchers found that treatment with AXL inhibitor bemcentinib downregulates ZEB1. Based on their findings, they concluded that AXL inhibitors could serve as a therapy to shift SARS-CoV-2 infected cells away from a mesenchymal phenotype.

Another recent publication indicated that AXL is a candidate receptor for SARS-CoV-2 that promotes infection of pulmonary and bronchial epithelial cells (Wang 2021). The tyrosine-protein kinase receptor AXL was found to specifically interact with the N-terminal domain of SARS-CoV-2 S. In both SARS-CoV-2 virus pseudotype and authentic SARS-CoV-2, overexpression of AXL in HEK293T cells was demonstrated to promote SARS-CoV-2 entry as efficiently as overexpression of ACE2, while knocking out AXL was shown to significantly reduces SARS-CoV-2 infection in H1299 pulmonary cells and in human primary lung epithelial cells. Soluble human recombinant AXL blocks SARS-CoV-2 infection in cells expressing high levels of AXL. The AXL expression level is well correlated with SARS-CoV-2 S level in bronchoalveolar lavage fluid cells from COVID-19 patients.

### REFERENCES:

Bhattacharyya S et al. (2013) Enveloped viruses disable innate immune responses in dendritic cells by direct activation of TAM receptors. Cell Host Microbe. 14(2): 136-147.

Bhatti Jasvinder ingh et al. (2020) Therapeutic Strategies in the Development of Anti-viral Drugs and Vaccines Against SARS-CoV-2 Infection. Molecular Neurobiology Springer. US, New York 57(11): 4856-4877.

US 2015/252370 A1

Canadas I et al. (2018) Tumor innate immunity primed by specific interferon-stimulated endogenous retroviruses. Nat Med. 24(8): 1143-1150.

Chen J et al. (2018) AXL promotes Zika virus infection in astrocytes by antagonizing type I interferon signaling. Nat Microbiol. 3: 302.

Cruz VH et al. (2019) Axl-mediated activation of TBK1 drives epithelial plasticity in pancreatic cancer. JCI Insight Apr 2 pii126117.

Dowall SD et al. (2016) Antiviral screening of multiple compounds against Ebola virus. Viruses, 8: 27.

Gay, C. M., K. Balaji and L. A. Byers (2017). Giving AXL the axe: targeting AXL in human malignancy. Br J Cancer 116(4): 415-423.

Hastings AK et al. (2019) Loss of the TAM receptor Axl ameliorates severe Zika virus pathogenesis and reduces apoptosis in microglia. iScience. 13: 339.

Huang MT (2015) Feedback regulation of IFN-alpha/beta signaling by Axl receptor tyrosine kinase modulates HBV immunity. Eur J Immunol, 45: 1696.

Lee AJ et al. (2019) Type I Interferon receptor on NK cells negatively regulates interferon-gamma production. Front Immunol. 10: 1261.

Meertens L. et al. (2012) The TIM and TAM families of phosphatidylserine receptors mediate dengue virus entry. Cell Host & Microbe, 12: 544.

Meertens L. et al. (2017) Axl mediates ZIKA virus entry in human glial cells and modulates innate immune responses. Cell Rep 18: 324.

Rothlin CV et al. (2007) TAM receptors are pleiotropic inhibitors of innate immune response. Cell. 131: 1124-1136.

Schoumacher M. and M. Burbridge (2017). Key Roles of AXL and MER Receptor Tyrosine Kinases in Resistance to Multiple Anticancer Therapies. Curr Oncol Rep 19(3): 19.

Sharif MN et al. (2006) Twist mediates suppression of inflammation by type I IFNs and Axl. J Exp Med 203(8): 1891-1901.

Shibata T et al. (2014) Axl receptor blockade ameliorates pulmonary pathology resulting from primary viral infection and viral exacerbation of asthma. J Immunology. 192: 3569.

Shimojima M et al. (2006) Tyro3 family-mediated cell entry of Ebola and Marburg viruses. J Virol 80: 10109.

Strange DP et al. (2019) Axl promotes Zika virus entry and modulates the antiviral state of human Sertoli cells. mBio. 10: e01372.

Wium, M et al. (2018). The Dual Role of TAM Receptors in Autoimmune Diseases and Cancer: An Overview. Cells 7(10): pii E166.

Bhattacharyya S et al. (2013) Enveloped viruses disable innate immune responses in dendritic cells by direct activation of TAM receptors. Cell Host Microbe. 14(2): 136-147.

Bouhaddou M et al. (2020) The Global Phosphorylation Landscape of SARS-CoV-2 Infection. Cell. 182: 685-712.

Wang S et. al. (2020) AXL Promotes SARS-CoV-2 Infection of Pulmonary and Bronchial Epithelial Cells" https://doi.org/10.21203/rs.3.rs-35387/v1.

Stewart et al. (2020) SARS-CoV-2 infection induces EMT-like molecular changes, including ZEB1-mediated repression of the viral receptor ACE2, in lung cancer models, doi:10.1101/2020.05.28.122291. Preprint.

Wang et. al. (2021) AXL is a candidate receptor for SARS-CoV-2 that promotes infection of pulmonary and bronchial epithelial cells. Cell Research (2021) 0:1-15; https://doi.org/10.1038/s41422-020-00460-y.

### SUMMARY

Provided herein are antiviral therapies using a potent and selective AXL inhibitor. In particular, the AXL inhibitor may be used for preventing or treating infections of pulmonary and bronchial epithelial cells. Significantly, because AXL plays roles in viral entry as well as regulating cytokine production, viral replication and EMT, the AXL inhibitor described herein is capable of early disease intervention by lowering viral replication and inhibiting viral entry, thereby forestalling potential development of severe respiratory symptoms or other organ decompensation.

Specifically, the AXL inhibitor acting as an antiviral agent is 3-(5-(cyclopropylmethyl)-1,3,4-oxadiazol-2-yl)-5-(1-(piperidin-4-yl)-1H-pyrazol-4-yl)pyridin-2-amine ("SLC-391").

In various embodiments, the viral infection is caused by respiratory pathogens such as coronaviruses, including those viruses that infect humans and non-human animals, such as betacoronovirus (e.g., SARS and MERS).

In various embodiments, the anti-viral agent (SLC-391) is administered to a patient that is symptomatic for a viral infection. In other embodiments, the anti-viral agent (SLC-391) is administered to an asymptomatic patient that is tested positive for a viral infection.

In other embodiments, the anti-viral agent (SLC-391) is co-administered with a second anti-infective agent, e.g. interferon, ribivarin, and the like.

### BRIEF DESCRIPTION OF THE FIGURES

FIG. 1 shows the inhibitory effect of SLC-391 on SARS-CoV-2 infection of Vero E6 cells.
FIG. 2 shows a neutralization curve of SLC-391 in the letiviral-based SARS-CoV-2 pseudovirus neutralization assay.
FIG. 3 demonstrates the anti-viral effect against human influenza virus as evaluated by cytopathic effect (CPE).

### DETAILED DESCRIPTION

Various embodiments of the present disclosure are directed to the use of a TAM family kinase inhibitor (e.g., an AXL inhibitor) as an anti-infective agent for the prevention or treatment of infectious diseases, including infection of pulmonary and bronchial epithelial cells. In particular, the AXL inhibitor (SLC-391) is effective against severe acute respiratory syndrome (SARS) caused by coronaviruses such as SARS-CoV-1, SARS-CoV-2, and MERS-CoV.

### AXL Inhibitor SLC-391

Selective AXL inhibition provides a viable therapy for treating diseases caused by viral pathogens. The AXL inhibitor (SLC-391) of the present disclosure has the following chemical structure:

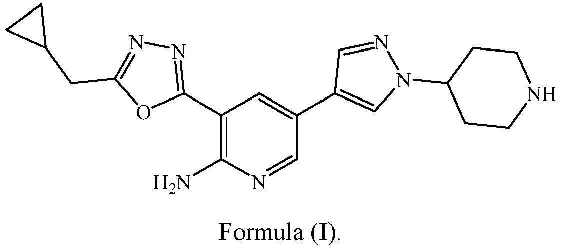

SLC-391 is a selective and potent small molecule AXL inhibitor, potential first-in-class with desirable potency and pharmaceutical properties. SLC-391 is an oral AXL kinase inhibitor with an IC₅₀ in the nanomolar (nM) range that has demonstrated in vitro and in vivo activity in hematologic and solid tumors. SLC-391 and its pharmaceutically acceptable forms (e.g., salt, solvate, hydrate or prodrug) are described in more detail in WO 2015/081257, which is incorporated herein by reference in its entirety.

SLC-391 is currently being evaluated in a Phase I, open-label, dose-escalation and dose expansion study of the safety and pharmacokinetics after oral administration to subjects with solid tumors (SLC-391-101). The nonclinical and safety pharmacology studies supporting the Phase I trial, along with access to clinical drug product already known to be well tolerated in human subjects with excellent exposure, makes SLC-391 uniquely poised to enter the clinic for evaluation as an anti-viral therapeutic, and investigated as a potential treatment for hospitalized COVID-19 patients.

### A. In Vitro Pharmacology

SLC-391 is a potent and specific AXL inhibitor (IC₅₀ for AXL is 9.6 nM versus 42.3 nM for TYRO3 and 63.5 nM for MER). SLC-391 has also demonstrated good selectivity for TAM kinases compared with other tyrosine kinases. In an in vitro BaF3 cell assay, SLC-391 was screened against a panel of 93 receptor tyrosine kinases and the results showed that SLC-391 at 100 nM inhibits only 4 receptor tyrosine kinases by >60 % (AXL [69%], MER [61 %], VEGFR1 [64%], FGFR3 [64%]). The selectivity of SLC-391 was further evaluated against a panel of 320 protein kinases from the human kinome using a radiometric phosphotransferase assay. The results indicated a good selectivity profile for SLC-391 and the following kinases were inhibited to a similar degree to that of AXL (IC50 values): c-MET (12 nM), RET (28 nM), ROS1 (23 nM), EPHA6 (45 nM), ALK1 (31 nM), NUAK1 (62 nM), and Aurora C (51 nM). In addition, the IC50 of SLC-391 for inhibition of Gas6-induced phosphorylation of Akt, indicative of AXL activity, was 0.29 µM in a human NSCLC cell line, A549.

SLC-391 has been shown to exhibit antiproliferative activity in cell lines of various origins in which AXL is abundantly expressed, including lung, breast, and hematopoietic lineages. The IC50 values for cellular proliferation activity ranged from 1.05 µM (4T1) to 3.45 µM (K562). SLC-391 also demonstrated concentration-dependent impairment of anchorage-independent cell proliferation, with complete inhibition observed at 1 µM in A549 cells. Although only a modest effect (less than 40% reduction at 1 µM) on cell migration was seen in A549, SLC-391 impeded cell invasion at concentrations much lower than the IC50 value for antiproliferation; SLC-391 at 0.2 µM inhibited A549 cell invasion by approximately 75%.

### B. In Vivo Pharmacology

Significant in vivo pharmacology studies for SLC-391 have been completed in support of its oncology indication. The bioavailability of SLC-391 in vivo when administered orally (PO) (twice-daily [BID] and once-daily [QD]) were studied. The biologically effective dose of SLC 391 was considered to be between 50 to 75 mg/kg BID with tumor growth inhibition (TGI) ranging from 60% to 67%, respectively. Oral administration of SLC-391 also resulted in a 41% reduction in levels of phosphorylated Akt in tumor xenograft samples.

### C. Safety Pharmacology

Safety pharmacology, nonclinical pharmacokinetics (PK) and metabolism studies have been completed for SLC-391 in support of the current Phase 1 clinical program. In a standard hERG assay, SLC-391 did not exhibit hERG inhibition at the tested concentrations with a half-maximal inhibitory concentration (IC50) determined to be more than 30 µM. The IC50 of the reference standard dofetilide was 20 nM which is within the range reported in the literature.

In a 28-day oral gavage toxicology study in Beagle dogs (males and females), SLC-391 had no observable effects on the morphology of the P-QRS-T complexes as well as the quantitative measurements of the PR interval, QT, and QTc intervals, or QRS complex duration. There were no effects on heart rate (HR) and no SLC-391-related effects on respiratory parameters. In a 28-day oral gavage toxicology study in Sprague-Dawley rats (males and females), there were no SLC-391 related adverse central nervous system (CNS) effects based on a functional observation battery evaluation.

Single dose PK studies were conducted in 3 species (CD-1 mouse, Sprague-Dawley rat, and Beagle dog) using PO and IV administration in order to determine the bioavailability of SLC-391. In addition, TK was evaluated after single and repeated oral dosing as part of the Good Laboratory Practice (GLP) 28-day oral toxicology studies in rats and dogs.

In genotoxicity screening studies, SLC-391 was negative in Salmonella tester strains TA98 and TA100 (Ames screening test) with and without liver S9 metabolic activation. In the Flowscreen assay, SLC-391 was classified as potentially genotoxic with an aneugenic mechanism of action and a potential genotoxic response was observed in the in vivo rat micronucleus assay at the highest dose of 70 mg/kg/day, but not at 13 mg/kg/day.

All nonclinical toxicology studies have been completed for SLC-391 in support of the current Phase I clinical program. These include single-dose maximum tolerated dose (MTD) in rats and dogs, Non-GLP, repeated-dose studies in rats and dogs, and 28 Day GLP studies in rats and dogs. In the 28-day rat GLP toxicology study, the NOAEL was 30 mg/kg/day. The 70 mg/kg/day dose level (highest dose tested) is defined as the severe toxic dose for the purpose of calculating the starting clinical dose, for which the human equivalent dose (HED) equals 11.3 mg/kg. In the 28-day dog GLP toxicology study, 6 mg/kg/day was the HNSTD as defined in ICH S9, for which the HED equals 3.3 mg/kg. Since the dog HED (3.3 mg/kg) is lower than the rat HED (11.3 mg/kg), dogs appear to be more sensitive than rats to SLC-391 toxicity. This increased sensitivity may be related to higher oral bioavailability in dogs compared to rats as observed in PK studies.

### Therapeutic Uses of SLC-391

Various embodiments provide therapeutic uses of SLC-391, or any one of its pharmaceutically acceptable forms (e.g., salt, solvate, hydrate or prodrug), as an antiviral agent against an infection or a diseases caused by an infection in a subject. The terms "subject," and "patient" are used interchangeably herein, and refer to an animal, including, but not limited to, human and non-human primates, including simians and humans; rodents, including rats and mice; bovines; equines; ovines; felines; canines; and the like. "Mammal" means a member or members of any mammalian species, and includes, by way of example, canines; felines; equines; bovines; ovines; rodentia, etc. and primates, e.g., non-human primates, and humans. Non-human animal models, e.g., mammals, *e.g.* non-human primates, murines, lagomorpha, etc. may be used for experimental investigations.

One specific embodiment provides a compound of Formula (I) (*i.e.,* SLC-391) for use in preventing or treating a disease or an infection in a subject. In more specific embodiments, the infection is caused by coronavirus or influenza virus.

In some embodiments, the infection or disease is caused by a coronavirus. In more specific embodiments, the coronavirus is a SARS coronavirus (SARS-CoV) and/or a SARS-CoV-2. In an even more specific embodiment, the disease is COVID-19 caused by the 2019 novel coronavirus SARS-CoV-2.

In some embodiments, the disease caused by the coronavirus is a respiratory disease, which may include pneumonia (mild and severe), acute respiratory infections, severe acute respiratory infection (SARI), hypoxic respiratory failure, acute respiratory distress syndrome, sepsis, septic shock, or severe acute respiratory syndrome (SARS).

The symptoms of respiratory diseases may include mild symptoms from simple infections, such as fever, cough, sore throat, nasal congestion, fatigue, headache, and muscle pain. The symptoms for more severe infections (e.g., pneumonia, SARI) may include increased breathing frequency, severe respiratory failure or dyspnea, central cyanosis, drowsiness, unconsciousness or convulsion, gasp, etc. Elderly people and immunosuppressed people may have atypical symptoms.

In certain embodiments, the anti-infective agent (SLC-391) is administered to an asymptomatic subject who has tested positive for a coronavirus without showing symptoms.

In other embodiments, the anti-infective agent (SLC-391) is administered to a subject within 0-7 days, or within 0-5 days, or within 0-3 days, or within 0-1 day of testing positive for a coronavirus.

"Testing positive" means a definitive detection of the genetic material from a specific virus or the proteins or protein fragments on the surface of a specific virus.

Another embodiment provides a compound of Formula (I) (SLC-391) for use in inhibiting or reducing the replication or reproduction of a virus in mammalian cells of a subject. In particular, the cells are pulmonary and bronchial epithelial cells. In more specific embodiments, the virus is coronavirus and the compound of Formula (I) reduces the nucleic acid load of coronavirus in cells.

A further embodiment provides a compound of Formula (I) (SLC-391) for use in blocking SARS-CoV-2 spike protein from binding to its host receptor ACE2.

In other embodiments, the anti-viral agent (SLC-391) is co-administered with a second anti-infective agent, *e.g.* interferon, ribivarin, ritonavir, monoclonal antibodies, and the like.

In preferred embodiments, the compound of Formula (I) (SLC-391) is administered orally. In other embodiments, the compound of Formula (I) (SLC-391) is administered by intraperitoneal or intravenous administration.

### EXAMPLE 1

### ANTIVIRAL CELL-BASED ASSAY

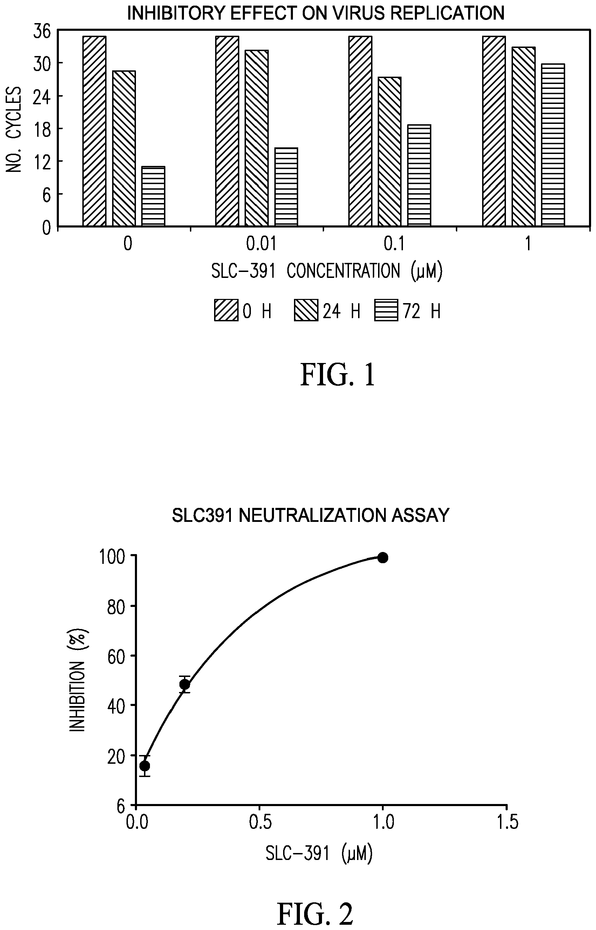

A valid cell-based assay was conducted to test SLC-391 on the inhibition of SARS-CoV-2 infection of Vero E6 cells using qPCR method. Fig. 1 summarizes the results, which demonstrate that SLC-391 was capable of inhibiting SARS-CoV-2 infection of Vero E6 cells as measured by the cycles required to replicate the viral RNA (the higher the number of cycles, the lower the infection). As shown, a high degree of viral inhibition was achieved up to 72 hours at a concentration of 1 µM. Even at 0.01 µM concentration, SLC-391 could effectively inhibit the viral infection for 24 hours. Fig. 1 further demonstrates a dose-dependent inhibition of the viral replication.

### EXAMPLE 2

### SLC-391 INHIBITS SARS-CoV-2 SPIKE PROTEIN BINDING TO ACE2 IN A PSEUDOVIRUS NEUTRALIZATION ASSAY

Method: The inhibitory activity of SLC391 for blocking SARS-CoV-2 spike protein from binding to its host receptor ACE2 was measured by using GenScript pseudovirus neutralization assay. SLC391 was serially diluted with Opti-MEM in duplicate, mixed with pseudovirus in an assay plate, and incubated at room temperature for 1 hour. Human ACE2 overexpressed CHO cells, CHO-hACE2 cells, were diluted to a concentration of 6 × 10⁶ cells/mL in DMEM complete growth medium and added to the SLC-391-pseudovirus mixtures. After incubation at 37°C and 5% CO₂ for 34 h, fresh DMEM complete growth medium was added and incubated for another 24 h. The growth medium was carefully removed. Freshly-made luciferase detection reagent was added immediately after medium removal and incubated at room temperature for 15 minutes. Luciferase activity (in relative light (RLU)) was measured. Percent neutralization was normalized considering uninfected cells as 100% neutralization and cells infected with pseudovirus alone as 0% neutralization.

Results: SLC391 SARS-CoV-2 neutralization assay is a lentiviral-based pseudovirus neutralization assay for detecting the potency of SLC-391 in neutralizing SARS-CoV-2 spike protein. The spike protein described in this assay is located on the surface of the lentivirus-based pseudovirus particles, so the pseudoviurs can bind to the ACE2 overexpressed by the CHO-hACE2 cells, simulating the fusion process of the coronavirus-cell membrane. After infection, the pseudovirus can express the carried luciferase gene in the cells. By measuring luciferase activity, the extent of pseudovirus infection can be inferred. In the neutralization assay tested, SLC-391 significantly decreased bioluminescence signal (Fig. 2). This demonstrates that SLC-391 is able to block the interaction of ACE2 and spike proteins and prevent the pseudovirus infection in a dose-dependent manner. At concentration of 1 µM, SLC-391 was capable of completely neutralize the SARS-CoV-2 pseudovirus binds to CHO-hACE2 cells and prevent its infection.

### EXAMPLE 3

### SLC-391 PROVIDES THE CYTOPROTECTION ON HUMAN FLU A INDUCED CYTOPATHIC EFFECT

Method: Inhibition of virus-induced cytopathic effects (CPE) and cell viability following human influenza virus (FluAPR834) replication in RPMI2650 cells was measured by a chemiluminescenct endpoint (CellTiterGlo). Cells (5 × 10⁵ cells per well) were seeded in 96-well flat-bottom tissue culture plates and allowed to adhere overnight at 37°C and 5% CO₂. Following incubation, diluted test compounds and virus diluted to a pre-determined titer to yield at least 50% cell killing at 4 days (Flu A) post-infection were added to the plate. Following incubation at 37°C, 5% CO₂ for 4 days, cell viability was measured using a CellTiterGlo. Percent reduction of the virus-infected wells and the percent cell viability of uninfected compound control wells were calculated to determine the EC₅₀ and TC50 values using four parameter curve fit analysis. The EC₅₀ was the concentration of test compound to inhibit CPE by 50%; The TC₅₀ was the concentration of test compound that caused 50% cell death in the absence of virus. The therapeutic index (TI) was calculated as TC₅₀ divided by EC₅₀.

Results: SLC-391 showed the cytoprotection on human Flu A induced cytopathic effect (CPE) in RPMI2650 cells. EC50 and TC50 were 0.14 µM and >10 µM respectively. The therapeutic index (TI) reached above 71.4.

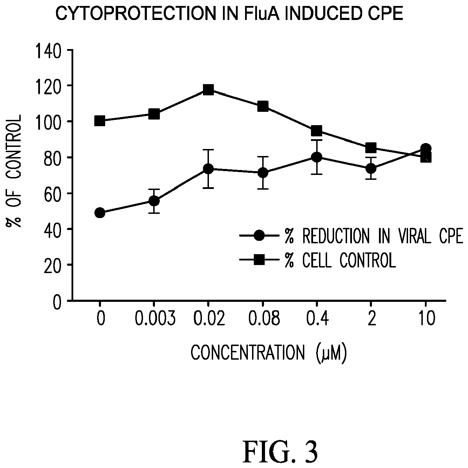

As shown in Fig. 3, the cytoprotection effects of SLC-391 on human influenza virus (Flu A) infection in RPMI2650 cells was evaluated. Cells were treated with Flu A in the presence or absence of various concentrations of SLC-391 at 37°C, 5% CO₂ for 4 days. SLC-391 induced cytotoxicity was evaluated without virus infection. Cell viability was measured by CellTiterGlo. The percent reduction in viral CPE and the percent of cell control was determined from cells without SLC-391 treatment.

### EXAMPLE 4

### SLC391 DEMONSTRATED VIRUS YIELD REDUCTION IN FLU A INFECTED RPMI2650 CELLS

Method: RPMI2650 cells were seeded at 5000 cells/well and incubated overnight for adherence to the 96 well microtiter plates. Following overnight incubation, cell culture medium was removed, and cell monolayers were washed one time with PBS. One hundred (100) µL/well assay medium was added to the plates. Supernatant samples at test concentration 10 µM, 0.2 µM and 0.003 µM from the Flu A cytoprotection assay (day 4 post-infection) were collected and each treatment concentration was pooled. The triplicate virus control supernatants were pooled. Samples were serially diluted in logarithmic increments in assay medium and added to the cells at 100 µL/well in quadruplicates. Following incubation at 37°C, 5% CO₂ for 4 days, cell viability staining with CellTiterGlo and TCID₅₀/mL were calculated. Fold shift in virus infectivity of SLC-391 treated virus was determined by comparison to untreated virus.

**Table 1; Effect of SLC-391 on influenza virus (Flu A) yield reduction assay**

| Concentration (uM) | TCID50/mL | Fold of virus reduction | CPE assay (% cell viability) |
|---|---|---|---|
| 10 | 731 | 952.9 | 80 |
| 0.4 | 9290 | 74.9 | 95 |
| 0.003 | 19999 | 34.8 | 100 |
| Virus Control | 696627 | --- | --- |

Result: SLC-391 demonstrated virus yield reduction in Flu A infected RPMI2650 cells. When SLC-391 at 10 µM, 0.4 µM and 0.003 µM test concentration, the fold of virus reduction reached 952.9, 74.9 and 34.8 respectively when compared with the virus control.

U.S. Patent Application No. 63/144,453, filed February 1, 2021, is cited.

From the foregoing it will be appreciated that, although specific embodiments of the disclosure have been described herein for purposes of illustration, various modifications may be made without deviating from the scope of the disclosure. Accordingly, the disclosure is not limited except as by the appended claims.

## Claims

1. A compound of Formula (I) for use in preventing or treating an infection or a disease caused by an infection, wherein the compound has the following structure:

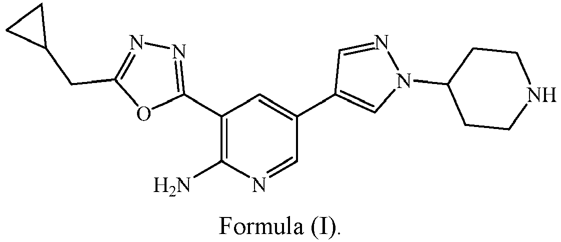

2. The compound for use according to claim 1 wherein the use comprises preventing or treating an infection caused by a coronavirus or an influenza virus.

3. The compound for use according to claim 1 or claim 2 wherein the coronavirus is SARS-CoV or SARS-CoV-2.

4. The compound of for use according to any one of claims 1-3 wherein the disease caused by an infection is a respiratory disease.

5. The compound for use according to any one of claims 1-4 wherein the disease is COVID 19.

6. The compound for use according to any one of claims 2-5 wherein the use comprises administering the compound of Formula (I) to an asymptomatic subject who has tested positive for the virus.

7. The compound for use according to any one of claims 2-5 wherein the use comprises administering the compound of Formula (I) to a subject within 0-7 days of testing positive for the virus.

8. A compound of Formula (I) for use in inhibiting or reducing replication or reproduction of a virus in cells of a subject, wherein the compound has the following structure:

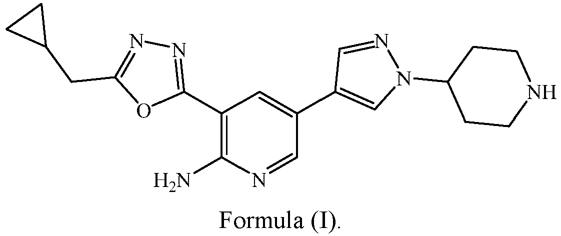

9. The compound for use according to claim 8 wherein the cells are pulmonary and bronchial epithelial cells.

10. The compound for use according to claim 8 or claim 9 wherein the virus is a coronavirus or an influenza virus.

11. The compound for use according to any one of claims 8-10 wherein the coronavirus is SARS-CoV or SARS-CoV-2.

12. The compound for use according to any one of claims 8-11 wherein the use comprises administering the compound of Formula (I) to an asymptomatic subject who has tested positive for the virus.

13. The compound for use according to any one of claims 8-11 wherein the use comprises administering the compound of Formula (I) to a subject within 0-7 days of testing positive for the virus.

14. A compound of Formula (I) for use in blocking SARS-CoV-2 spike protein from binding to its host receptor ACE2, wherein the compound has the following structure:

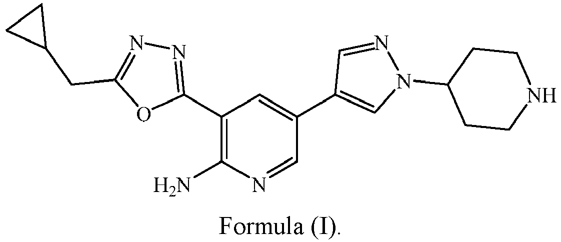

15. The compound for use according to claim 14 wherein the use comprises treating COVID 19.

## Patentansprüche

1. Verbindung der Formel (I) zur Verwendung bei der Vorbeugung oder Behandlung einer Infektion oder einer durch eine Infektion verursachten Erkrankung, wobei die Verbindung folgende Struktur aufweist:

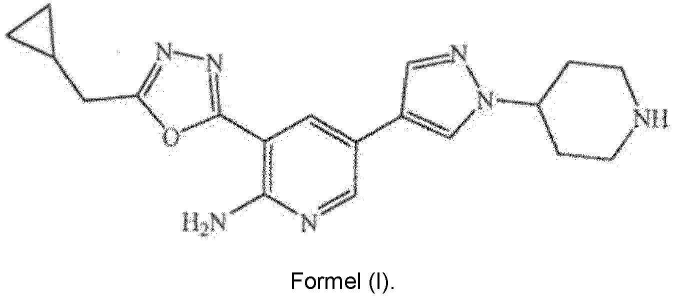

2. Verbindung zur Verwendung nach Anspruch 1, wobei die Verwendung das Vorbeugen oder Behandeln einer durch ein Coronavirus oder ein Influenzavirus verursachten Infektion umfasst.

3. Verbindung zur Verwendung nach Anspruch 1 oder 2, wobei es sich bei dem Coronavirus um SARS-CoV oder SARS-CoV-2 handelt.

4. Verbindung zur Verwendung nach einem der Ansprüche 1 bis 3, wobei es sich bei der durch eine Infektion verursachte Erkrankung um eine Atemwegserkrankung handelt.

5. Verbindung zur Verwendung nach einem der Ansprüche 1 bis 4, wobei es sich bei der Erkrankung um COVID 19 handelt.

6. Verbindung zur Verwendung nach einem der Ansprüche 2 bis 5, wobei die Verwendung das Verabreichen der Verbindung der Formel (I) an ein asymptomatisches Individuum, das positiv auf das Virus getestet wurde, umfasst.

7. Verbindung zur Verwendung nach einem der Ansprüche 2 bis 5, wobei die Verwendung das Verabreichen der Verbindung der Formel (I) an ein Individuum innerhalb von 0-7 Tagen nach positivem Testen auf das Virus umfasst.

8. Verbindung der Formel (I) zur Verwendung beim Hemmen oder Verringern der Replikation oder Reproduktion eines Virus in Zellen eines Individuums, wobei die Verbindung folgende Struktur aufweist:

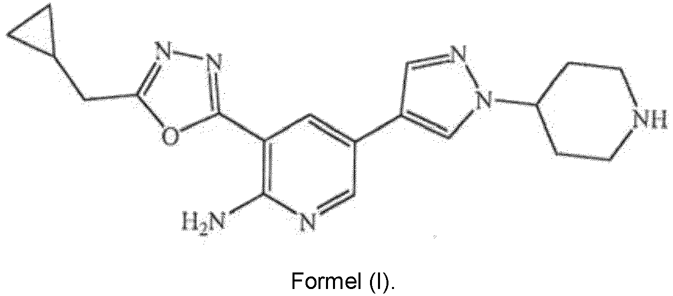

9. Verbindung zur Verwendung nach Anspruch 8, wobei es sich bei den Zellen um pulmonale und bronchiale Epithelzellen handelt.

10. Verbindung zur Verwendung nach Anspruch 8 oder 9, wobei es sich bei dem Virus um ein Coronavirus oder ein Influenzavirus handelt.

11. Verbindung zur Verwendung nach einem der Ansprüche 8 bis 10, wobei es sich bei dem Coronavirus um SARS-CoV oder SARS-CoV-2 handelt.

12. Verbindung zur Verwendung nach einem der Ansprüche 8 bis 11, wobei die Verwendung das Verabreichen der Verbindung von Formel (I) an ein asymptomatisches Individuum, das positiv auf das Virus getestet wurde, umfasst.

13. Verbindung zur Verwendung nach einem der Ansprüche 8 bis 11, wobei die Verwendung das Verabreichen der Verbindung der Formel (I) an ein Individuum innerhalb von 0-7 Tagen nach positivem Testen auf das Virus umfasst.

14. Verbindung der Formel (I) zur Verwendung beim Verhindern des Bindens eines SARS-CoV-2-Spike-Proteins an seinen Wirtsrezeptor ACE2, wobei die Verbindung folgende Struktur aufweist:

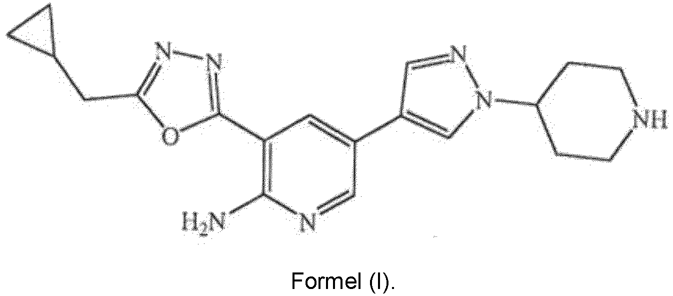

15. Verbindung zur Verwendung nach Anspruch 14, wobei die Verwendung das Behandeln von COVID 19 umfasst.

## Revendications

1. Composé de formule (I) pour utilisation pour prévenir ou traiter une infection ou une maladie causée par une infection, dans lequel le composé présente la structure suivante :

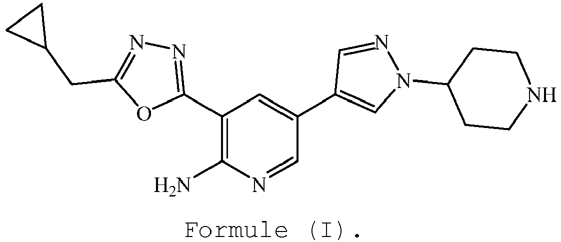

2. Composé pour utilisation selon la revendication 1, dans lequel l'utilisation comprend la prévention ou le traitement d'une infection causée par un coronavirus ou un virus de la grippe.

3. Composé pour utilisation selon la revendication 1 ou la revendication 2, dans lequel le coronavirus est le SARS-CoV ou le SARS-CoV-2.

4. Composé pour utilisation selon l'une quelconque des revendications 1 à 3, dans lequel la maladie provoquée par une infection est une maladie respiratoire.

5. Composition pour utilisation selon l'une quelconque des revendications 1 à 4, dans laquelle la maladie est la COVID 19.

6. Composé pour utilisation selon l'une quelconque des revendications 2 à 5, dans lequel l'utilisation comprend l'administration du composé de formule (I) à un sujet asymptomatique qui a été testé positif pour le virus.

7. Composé pour utilisation selon l'une quelconque des revendications 2 à 5, dans lequel l'utilisation comprend l'administration du composé de formule (I) à un sujet dans les 0 à 7 jours suivant le test positif pour le virus.

8. Composé de formule (I) pour utilisation pour inhiber ou réduire la réplication ou la reproduction d'un virus dans des cellules d'un sujet, dans lequel le composé présente la structure suivante :

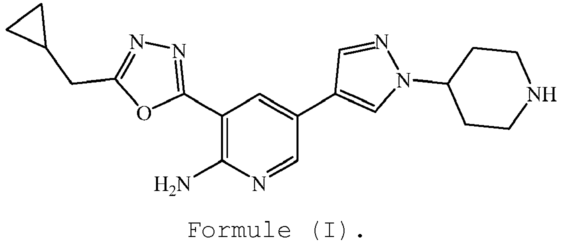

9. Composé pour utilisation selon la revendication 8 dans lequel les cellules sont des cellules épithéliales bronchiques ou pulmonaires.

10. Composé pour utilisation selon la revendication 8 ou la revendication 9 dans lequel le virus est un coronavirus ou un virus de la grippe.

11. Composé pour utilisation selon l'une quelconque des revendications 8 à 10, dans lequel le coronavirus est le SARS-CoV ou le SARS-CoV-2.

12. Composé pour utilisation selon l'une quelconque des revendications 8 à 11, dans lequel l'utilisation comprend l'administration du composé de formule (I) à un sujet asymptomatique qui a été testé positif pour le virus.

13. Composé pour utilisation selon l'une quelconque des revendications 8 à 11, dans lequel l'utilisation comprend l'administration du composé de formule (I) à un sujet dans les 0 à 7 jours suivant le test positif pour le virus.

14. Composé de formule (I) pour utilisation pour empêcher la protéine de spicule de SARS-CoV-2 de se lier à son récepteur hôte ACE2, dans lequel le composé présente la structure suivante :

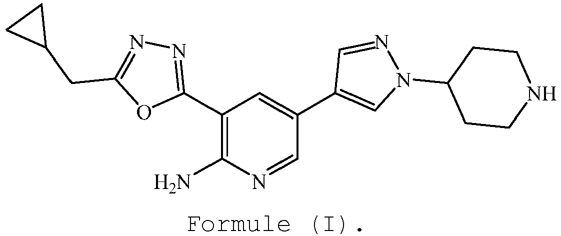

15. Composé pour utilisation selon la revendication 14, dans lequel l'utilisation comprend un traitement du COVID 19.
